# EUROPEAN PATENT APPLICATION

(11) **EP 1 361 266 A1**
(43) Date of publication of application: **12.11.2003**
(21) Application number: 01273199.8
(22) Date of filing: 27.12.2001
(51) Int. Cl.: C12N 5/06, C12N 5/22, C12N 15/09, C12N 15/12

(54) **TRANSPLANTATION CELL KITS FOR RE-MYELINATION IN NERVE CELLS**

(30) Priority: 09.01.2001 JP 2001001904
(71) Applicant: JAPAN SCIENCE AND TECHNOLOGY CORPORATION, Kawaguchi-shi, Saitama 332-0012 (JP)
(72) Inventor: TAMAMAKI, Nobuaki, Sakai-shi, Osaka 599-8124 (JP)
(74) Representative: Owen, Deborah Jane
(86) International application number: JP0111564
(87) International publication number: WO02055677

(57) **Abstract**

This invention provides cell kits characterized by comprising oligodendrocytes or oligodendrocyte precursors, and cells producing a substance promoting the migration of these cells. The cell kits can be used in re-myelination treatment of a demyelinated central nerve system (a spinal cord, a brain, an optic nerve and the like).]

## Description

### THCHNICAL FIELD

The invention of this application relates to cell kits. More specifically, the invention of this application relates to cell kits as transplantation cells which kits are used for re-myelination treatment of a demyelinated central nerve system (a spinal cord, a brain, an optic nerve and the like).

### BACKGROUND ART

Living bodies have themselves curability by which to repair damages. That is, bacterial infection from a damaged site is prevented, and great tissue destruction accompanied by an inflammatory reaction is caused in this site and also in an undamaged area to promote regeneration of a tissue from thereoutside. Accordingly, in case of a cut, a first injury is linear, but a scar tissue after healing is rendered in a facial state having a thickness.

In case of a damage of a central nerve system, such as a damage of a spinal cord, nerve cells or oligodendrocytes are, once lost, little regenerated or supplied, and no natural healing occurs. Nevertheless, in the damage of the spinal cord or the like, the same reaction as in other regeneratable tissues occurs, and inflammation or degeneration takes place in the vicinity of a damaged site. With respect to the myelin sheath, demyelination occurs in plural segments to inhibit transmission of neural information.

A large amount of an anti-inflammatory agent has been so far administered for inhibiting such degeneration and demyelination phenomena of the spinal cord. Further, drugs for suppressing cell death of cells generating the myelin sheath have been explored. As a result, there is an example in which good prognosis is obtained by preventing an indirect damage of the spinal cord from being widen via an inflammatory reaction from the area of the damaged spinal cord caused by a direct external force or the like.

However, the demyelination due to disorders or diseases, which has been once widened, cannot be recovered by natural curability. Accordingly, for re-myelinating the demyelinated region, attempts have been made to inject (transplant) cells (oligodendrocytes or oligodendrocyte precursors) for myelinating axons into the demyelinated region. Nevertheless, there is no successful example. There is a recent report stating that a re-myelination area was widening by 8 mm (Proc. Nat. Acad. Sci. USA 96: 15256-15261, 1999). However, the distance of 8 mm, which is less than a short diameter of the human spinal cord, and this is far from an object to recover a function by re-myelinating a demyelinated region.

As mentioned above, the attempts of re-myelination by transplantation of oligodendrocytes or oligodendrocyte precursors have not been successful, nor has there been any possibility of success. The reason is that wide-ranging myelination is impossible even by transplantation of oligodendrocytes.

Under the foregoing circumstances, the invention of this application has been made, and it aims to provide, by solving the problems associated with the related art, transplantation cell kits capable of re-myelinating axons in the wide range.

### DISCLOSURE OF THE INVENTION

This application provides, as the invention for solving the foregoing problems, cell kits characterized by comprising:
A: oligodendrocytes or oligodendrocyte precursors; and
B: cells producing a substance promoting the migration of cells A.

In the cell kits, a preferable embodiment is that cells A and cells B are both cells derived from humans or derived from mammals close to humans.

Further, a preferable embodiment is that cells A are derived from humans, and cells B are derived from animals close to humans, or cells A are derived from animals close to humans, and cells B are derived from humans.

Still further, a preferable embodiment is that cells A are recombinant cells using an animal gene that expresses a receptor to a substance promoting the migration of cells A, and/or cells B are recombinant cells using an animal gene that produces a substance promoting the migration of cells A.

### BRIEF DESCRIPTION OF THE DRAWINGS

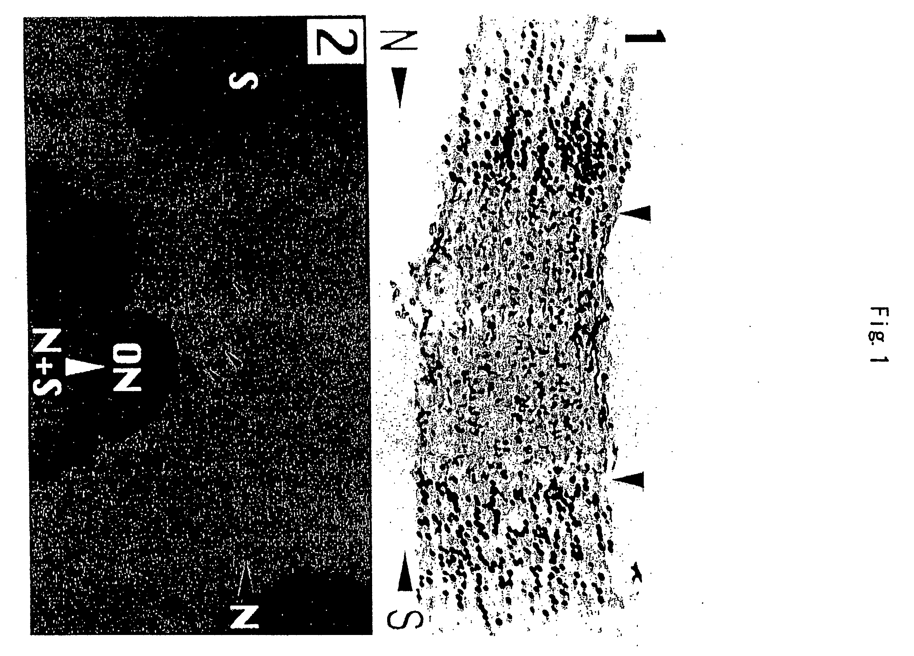

Fig. 1-1 is results provided by co-culturing a short optic nerve with COS cell lamps producing netrin and sema 3A respectively and wrapping the co-culture with a collagen gel. Optic nerve cells reacted with netrin migrated leftward, and cells reacted with sema 3A migrated rightward. Fig. 1-2 shows a distribution (small arrow) of oligodendrocyte precursors stained with anti-PLP antibody. The oligodendrocyte precursors migrate to evade netrin. N: netrin acting direction, S: sema 3A acting direction, N+S: acting direction in which netrin is mixed with sema 3A, ON: optic nerve fragment.

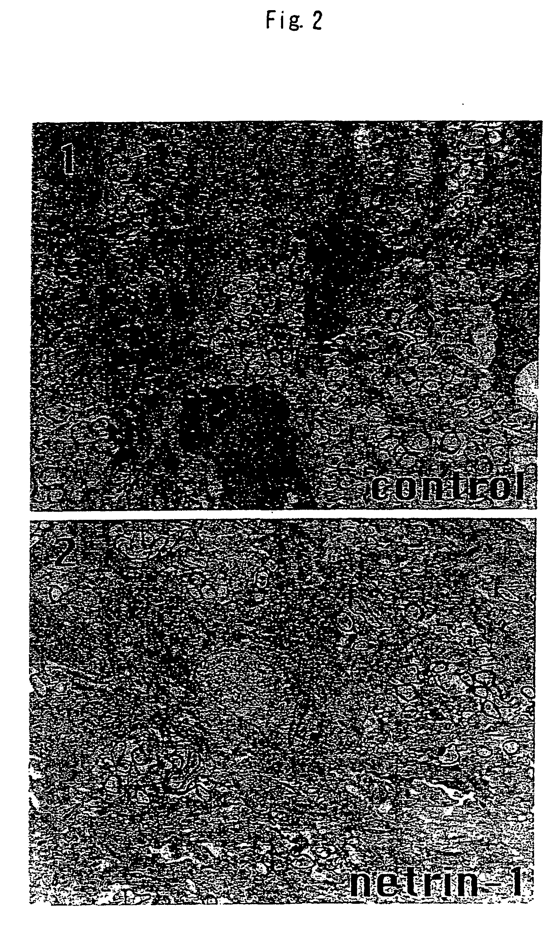

Fig. 2-1 is an electron micrograph showing a cross-section of an optic nerve of a 3-week-old normal rat. A circle surrounded with a black trim, which is observed in large quantities, is a myelinated optic nerve, and the black trim is a myelin sheath. Fig. 2-2 is an electron micrograph showing a cross-section of an optic nerve of a rat (3 weeks old) with a netrin expression vector introduced into retinal cells. A ratio of a myelinated optic nerve is extremely decreased.

### BEST MODE FOR CARRYING OUT THE INVENTION

The principle of the invention of this application is as follows. That is, intrinsic oligodendrocyte precursors of the brain are generated only from some limited regions of the fetal brain, and then migrate into respective sites where they differentiate into oligodendrocytes having a myelination ability (Neuron 19; 283-292, 1997; J. Neurosci. 18:8331-8343, 1998; Euro. J. Neurosci. 10 (suppl. 10): 338, 1998). In what direction the precursors migrate is determined by a concentration gradient of a substance which was secreted by various brain tissues and guides the precursor cell migration (a guidance cue). It is known that the precursors migrate in the concentration decreasing direction or in the concentration increasing direction. With what substance the precursors are reacted for migration is determined by a receptor that the precursors possess and by whether the receptor is reacted with the increase in concentration or with the decrease in concentration.

The invention of this application is cell kits comprising cells A (oligodendrocytes or their precursors) and cells B (cells producing a substance promoting the migration of cells A). These cells A and cells B are separately stored in solutions respectively, mixed just before transplantation, and subjected to transplantation in the central nerve along with the solutions thereof.

Examples of the substance promoting the migration include netrin, slit, sema 3A and the like. These substances act repulsively on cells A as will be later described in Examples, and have an effect to expel cells A to a remote site from the migration site. The cell kits of this invention are constructed from a combination of cells B producing these substances and cells A having a receptor to the substances promoting the migration which substances are produced from the cells B.

Cells A can be isolated from the fetal brain. Further, cells A can be obtained by differentiating neural stem cells with appropriate neurotrophins. Still further, oligodendrocytes produced from human ES cells or their precursors are also included therein.

In the thus-produced cells A, the type of the substance promoting the migration is determined depending on the type of the receptor thereof, or it can also be produced by gene recombination so as to express the receptor to a specific substance promoting the migration. Such a gene recombination can be performed by transfection of a eucaryotic cell expression vector carrying a polynucleotide (cDNA or the like) encoding a receptor protein to the substance promoting the migration. As the vector, known plasmid vectors or adenovirus vectors are available. As the gene for recombination, genes derived from humans, genes derived from animals close to humans, or genes obtained by partially modifying DNA sequences of these genes can be used.

Cells B are not particularly limited so long as they are cells producing the substance promoting the migration. However, in order to avoid immunological rejection, it is preferable to use human cells (for example, meningeal cells and the like) in which the cell kits are transplanted. Further, cells B are preferably cells that do not migrate in vivo.

Moreover, the cells B may be cells subjected to recombination for producing the substance promoting the migration which substance corresponds to the type of the receptor of cells A. It is advisable that such a recombination is performed by transfecting a eucaryotic cell expression vector carrying a polynucleotide (cDNA or the like) encoding the substance promoting the migration to primarily express the polynucleotide. As the gene for recombination, genes derived from humans, genes derived from animals close to humans, or genes obtained by partially modifying DNA sequences of these genes can be used.

A mixing ratio of cells A and cells B can be adjusted as required. When the amount of cells B is larger, the concentration gradient of the substance promoting the migration becomes higher and the cells reach to a distance, which enables re-myelination to a larger extent. Further, cells A are used in the minimum amount necessary for surely re-myelinating demyelinated axons. At this time, a level of cell division ability has to be taken into consideration. In case of using cells having high cell division ability, a small amount of cells A can be used in the transplantation.

### EXAMPLES

The invention of this application is illustrated specifically in more detail below by referring to Examples. However, the invention of this application is not limited by the following Examples.

### Example 1

An optic nerve was obtained from a newborn rat, and put into a culture medium. A limited site of the optic nerve in the culture medium was irradiated with ultraviolet light according to a known UV-thymine-dimer labeling method (Neurosci. Res. 35:241-251, 1999) to label a cell nucleus.

This optic nerve, a netrin-producing COS cell lamp and a sema 3A-producing COS cell lamp were arranged as shown in Fig. 1, and wrapped with a collagen gel.

After 3 hours of the incubation, the labeled cells were detected. As a result, it was observed that, as shown in Fig. 1-1, the cells reacted with netrin migrated in a remote site from the ultraviolet light irradiation region, and the cells reacted with sema 3A migrated in a remote site from the ultraviolet light irradiation region.

Subsequently, after the cells of the optic nerve were moved by the COS cell lamps, a marker substance PLP (Myelin Proteolipid Protein) of oligodendrocyte precursors was stained for visualization. Consequently, as shown in Fig. 1-2, it was identified that the cells reacted with netrin contained PLP (Development 128; 3321-3330, 2001).

From the foregoing results, it was identified that the presence of the cells producing netrin and sema 3A enables the artificial migration of the oligodendrocyte precursors.

### Example 2

A netrin expression vector was injected into an eyeball of a newborn rat, and introduced into cells inside the retina by an electroporation method. After this rat was grown to be 3 weeks old, the optic nerve was recovered after fixation with a fixative containing formalin and glutaraldehyde, and observed with an electron microscope. The results were as shown in Fig. 2. When netrin was expressed, as compared to the control, the number of myelinated fibers per unit area was small and myelination was inhibited significantly in the optic nerve in the vicinity of the eyeball.

From this result, it was identified that the artificial expression of netrin inhibits the myelination with the oligodendrocyte precursors and this inhibition is attributed to the promotion of the repulsive migration of the oligodendrocyte precursors with netrin.

### INDUSTRIAL APPLICABILITY

As has been thus far described in detail, this application provides the transplantation cell kits capable of re-myelinating the cells of the demyelinated central nerve system in the wide range. The cell kits promote the useful re-myelination treatment of the demyelinated central nerve system (a spinal cord, a brain, an optic nerve and the like).

## Claims

1. Cell kits **characterized by** comprising the following cells:
A: oligodendrocytes or oligodendrocyte precursors; and
B: cells producing a substance promoting the migration of cells A.

2. The cell kits of claim 1, wherein cells A and cells B are both cells derived from humans.

3. The cell kits of claim 1, wherein cells A and cells B are derived from mammals close to humans.

4. The cell kits of claim 1, wherein cells A are derived from humans, and cells B are derived from animals close to humans.

5. The cell kits of claim 1, wherein cells A are derived from animals close to humans, and cells B are derived from humans.

6. The cell kits of any of claims 1 to 5, wherein cells A are recombinant cells using an animal gene that expresses a receptor to a substance promoting the migration of cells A.

7. The cell kits of claim 6, wherein the animal gene is a gene derived from humans or animals close to humans, or a gene obtained by partially modifying DNA sequences of these genes.

8. The cell kits of any of claims 1 to 6, wherein cells B are recombinant cells using an animal gene that produces a substance promoting the migration of cells A.

9. The cell kits of claim 8, wherein the animal gene is a gene derived from humans or animals close to humans, or a gene obtained by partially modifying DNA sequences of these genes.
